(19) Europäisches Patentamt
European Patent Office
Office européen des brevets

(11) Veröffentlichungsnummer: **0 003 586**
**A1**

(12) **EUROPÄISCHE PATENTANMELDUNG**

(21) Anmeldenummer: **79100332.0**

(22) Anmeldetag: **05.02.79**

(51) Int. Cl.²: **C 07 C 59/26**, C 07 C 69/67, A 01 N 9/26

(30) Priorität: **13.02.78 DE 2805982**

(43) Veröffentlichungstag der Anmeldung: **22.08.79 Patentblatt 79/17**

(84) Benannte Vertragsstaaten: **BE CH DE FR GB IT NL**

(71) Anmelder: **Bayer Aktiengesellschaft, Zentralbereich Patente,Marken und Lizenzen Bayerwerk, D-5090 Leverkusen 1 (DE)**

(72) Erfinder: **Förster, Heinz, Dr., Am Eckbusch 47, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert Rudolf, Dr., Hahnenweg 5, D-5000 Köln 80 (DE)**

(54) **Phenoxyessigsäure-Derivate, Verfahren zu ihrer Herstellung und ihre Verwendung als Herbizide.**

(57) Neue Phenoxyessigsäure-Derivate der Formel

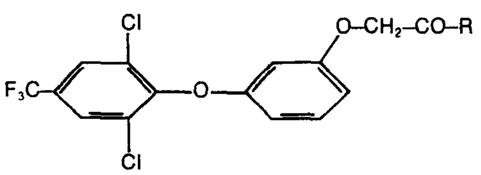

(I)

in welcher

R für Hydroxy, gegebenenfalls durch Alkoxy, Alkylthio, Carbalkoxy, Phenylmethylaminocarbonyl und/oder Halogen substituiertes Alkoxy, Amino, Hydrazino, gegebenenfalls durch Alkoxy oder Dialkylamino substituiertes Alkylamino, Dialkylamino, Alkenylamino, Arylamino oder N-Aryl-N-alkyl-amino, wobei der Arylrest jeweils durch Alkyl, Alkoxy, Carbalkoxy, Halogen und/oder Nitro substituiert sein kann, ferner für einen gegebenenfalls durch Alkyl substituierten, über Stickstoff gebundenen gesättigten heterocyclischen Ring mit 5 oder 6 Ringgliedern, oder für den Rest der Formel

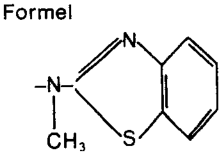

steht,

mehrere Verfahren zu ihrer Herstellung sowie die Verwendung dieser neuen Phenoxyessigsäure-Derivate als Herbizide.

EP 0 003 586 A1

BAYER AKTIENGESELLSCHAFT
Zentralbereich
Patente, Marken und Lizenzen

5090 Leverkusen, Bayerwerk
Dü/kl/Kü

Phenoxyessigsäure-Derivate, Verfahren zu ihrer Herstellung und ihre Verwendung als Herbizide

Die vorliegende Erfindung betrifft neue Phenoxy-essigsäure-Derivate, mehrere Verfahren zu ihrer Herstellung sowie ihre Verwendung als Herbizide.

Es ist bereits bekannt geworden, daß 3-(4-Chlorphenoxy)-α-phenoxy-propionsäure-äthylester zur Bekämpfung von Unkraut geeignet ist (vgl. DT-OS 2 716 189). Die Wirksamkeit dieses Stoffes ist jedoch, insbesondere bei niedrigen Dosierungen, nicht immer ganz befriedigend.

Gegenstand dieser Erfindung sind neue Phenoxyessigsäure-Derivate der Formel

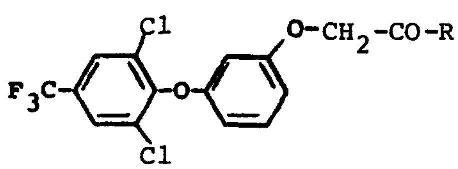

(I)

in welcher

R für Hydroxy, gegebenenfalls durch Alkoxy, Alkylthio, Carbalkoxy, Phenylmethylaminocarbonyl und/oder Halogen substituiertes Alkoxy, Amino, Hydrazino, gegebenenfalls durch Alkoxy oder Dialkylamino substituiertes Alkylamino, Dialkylamino, Alkenylamino, Arylamino oder N-Aryl-N-alkyl-amino, wobei der Arylrest jeweils durch Alkyl, Alkoxy, Carbalkoxy, Halogen und/oder Nitro substituiert sein kann, ferner für einen gegebenenfalls durch Alkyl substituierten, über Stickstoff gebundenen gesättigten heterocyclischen Ring mit 5 oder 6 Ringgliedern, oder für den Rest der Formel

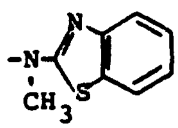

steht.

Gegenstand der Erfindung sind auch Verfahren zur Herstellung der Phenoxyessigsäure-Derivate der Formel (I). So erhält man

a) die Verbindungen der Formel (I), indem man das Phenoxyessigsäurechlorid der Formel

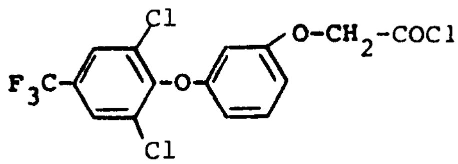

(II)

mit Verbindungen der Formel

HR (III)

in welcher

R die oben angegebene Bedeutung hat,

gegebenenfalls in Gegenwart eines Säureakzeptors sowie gegebenenfalls in Gegenwart eines Verdünnungsmittels umsetzt,

oder

b) diejenige Verbindung der Formel (I), in der R für Methoxy steht, indem man den Diphenyläther der Formel

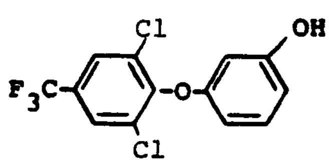

(IV)

mit Bromessigsäure-methylester der Formel

$$Br-CH_2-COOCH_3 \qquad (V)$$

in Gegenwart eines Säureakzeptors sowie in Gegenwart eines Verdünnungsmittels umsetzt,

oder

c) diejenige Verbindung der Formel (I), in der R für Hydroxyl steht, indem man die Verbindung der Formel

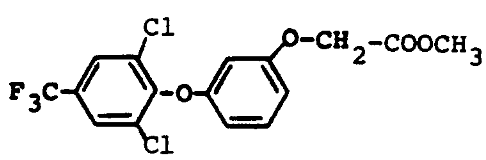

(I')

mit Basen in Gegenwart eines Verdünnungsmittels behandelt und anschließend ansäuert,

d) diejenigen Verbindungen der Formel (I), in denen R für Alkoxy mit mehr als einem Kohlenstoffatom steht, indem man die Verbindung der Formel

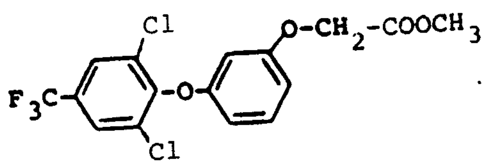

(I')

mit Alkoholen der Formel

$$HOR^1 \quad (VI)$$

in welcher

$R^1$ für Alkyl mit mehr als einem Kohlenstoffatom steht,

gegebenenfalls in Gegenwart eines Alkoholates sowie gegebenenfalls in Gegenwart eines Verdünnungsmittels umsetzt.

Gegenstand dieser Erfindung ist ferner die Verwendung von Phenoxyessigsäure-Derivaten der Formel (I) als Herbizide.

Überraschenderweise besitzen die erfindungsgemäßen Phenyloxyessigsäure-Derivate sehr gute herbizide Eigenschaften. So eignen sie sich z.B. zur selektiven Bekämpfung von Unkraut in verschiedenen Kulturen. Vor allem zeigen sie eine ausgezeichnete Wirkung gegen schwer bekämpfbare Unkräuter, wie z.B. Galium.

Die erfindungsgemäßen Stoffe stellen somit eine Bereicherung der Technik dar.

Die erfindungsgemäßen Phenoxy-essigsäure-Derivate sind durch die Formel (I) allgemein definiert. In der Formel (I) steht R vorzugsweise für Hydroxyl oder geradkettiges oder verzweigtes Alkoxy mit 1 bis 4 Kohlenstoffatomen, wobei jeder dieser Alkoxyreste ein- oder mehrfach, gleichartig oder verschieden substituiert sein kann. Als Substituenten kommen hierbei vorzugsweise in Frage Alkoxy mit 1 bis 4, insbesondere 1 bis 2 Kohlenstoffatomen, Alkylthio mit 1 bis 4, insbesondere 1 bis 2 Kohlenstoffatomen, Carbalkoxy mit 1 bis 4, insbesondere 1 bis 2 Kohlenstoffatomen in der Alkoxygruppe, Phenylmethylaminocarbonyl sowie Chlor und Brom. Weiterhin steht R vorzugsweise für Amino, Hydrazino, gegebenenfalls durch Alkoxy mit 1 bis 4 Kohlenstoffatomen oder durch Dialkylamino mit 1 bis 4 Kohlenstoffatomen je Alkylgruppe substituiertes Alkylamino mit 1 bis 4 Kohlenstoffatomen, Dialkylamino mit 1 bis 4 Kohlenstoffatomen je Alkylgruppe, Alkenylamino mit bis zu 4 Kohlenstoffatomen oder für Phenylamino bzw. N-Phenyl-N-alkyl-amino mit 1 bis 4 Kohlenstoffatomen in der Alkylgruppe, wobei der Phenylrest jeweils substituiert sein kann durch Alkyl mit 1 bis 4 Kohlenstoffatomen, Alkoxy mit 1 bis 4 Kohlenstoffatomen, Carbalkoxy mit 1 bis 4 Kohlenstoffatomen in der Alkoxygruppe, Fluor, Chlor, Bom und/oder Nitro. Darüber hinaus steht R vorzugsweise für einen gegebenenfalls durch Methyl substituierten, über Stickstoff gebundenen gesättigten heterocyclischen Ring mit 5 oder 6 Ringgliedern, wobei in dem Ring außer dem an die Carbonylgruppe gebundenen Stickstoffatom noch ein weiteres Stickstoff- oder Sauerstoffatom enthalten sein kann. Als Beispiele für derartige Heterocyclen seien genannt Piperidin, 2-Methyl-piperidin, Pyrrolidin, Piperazin, Pyrazolidin und Morpholin. Schließlich steht R vorzugsweise für den Rest der Formel

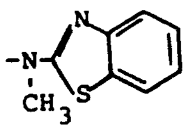 .

Als Beispiele für erfindungsgemäße Phenoxyessigsäure-Derivate der Formel (I) seien im einzelnen genannt:

3-(2,6-Dichlor-4-trifluormethyl-phenoxy)-phenoxy-essigsäure
3-(2,6-Dichlor-4-trifluormethyl-phenoxy)-phenoxy-essigsäure-methylester
3-(2,6-Dichlor-4-trifluormethyl-phenoxy)-phenoxy-essigsäure-äthylester
3-(2,6-Dichlor-4-trifluormethyl-phenoxy)-phenoxy-essigsäure-isopropylester
3-(2,6-Dichlor-4-trifluormethyl-phenoxy)-phenoxy-essigsäure-n-butylester
3-(2,6-Dichlor-4-trifluormethyl-phenoxy)-phenoxy-essigsäure-(2-methoxy)-äthylester
3-(2,6-Dichlor-4-trifluormethyl-phenoxy)-phenoxy-essigsäure-(2-methylthio)-äthylester
3-(2,6-Dichlor-4-trifluormethyl-phenoxy)-phenoxy-essigsäure-(2-äthoxycarbonyl)-äthylester

3-(2,6-Dichlor-4-trifluormethyl-phenoxy)-phenoxy-essigsäure-(2-phenylmethyl-aminocarbonyl)-äthylester

3-(2,6-Dichlor-4-trifluormethyl-phenoxy)-phenoxy-essigsäure-(2-chlor)-äthylester

3-(2,6-Dichlor-4-trifluormethyl-phenoxy)-phenoxy-essigsäure-methylamid

3-(2,6-Dichlor-4-trifluormethyl-phenoxy)-phenoxy-essigsäure-(2-methoxy)-äthylamid

3-(2,6-Dichlor-4-trifluormethyl-phenoxy)-phenoxy-essigsäure-(2-dimethylamino)äthylamid

3-(2,6-Dichlor-4-trifluormethyl-phenoxy)-phenoxy-essigsäure-dimethylamid

3-(2,6-Dichlor-4-trifluormethyl-phenoxy)-phenoxy-essigsäure-buten-2-yl-amid

3-(2,6-Dichlor-4-trifluormethyl-phenoxy)-phenoxy-essigsäure-(4-nitro-phenyl)-methylamid

3-(2,6-Dichlor-4-trifluormethyl-phenyl)-phenoxy-essigsäure-piperidid

3-(2,6-Dichlor-4-trifluormethyl-phenoxy)-phenoxy-essigsäure-pyrrolidid

3-(2,6-Dichlor-4-trifluormethyl-phenoxy)-phenoxy-essigsäure-piperazid

3-(2,6-Dichlor-4-trifluormethyl-phenoxy)-phenoxy-essigsäure-pyrazolidid

3-(2,6-Dichlor-4-trifluormethyl-phenoxy)-phenoxy-essigsäure-morpholid

3-2,6-Dichlor-4-trifluormehtyl-phenoxy)-phenoxy-essigsäure-(2-benzothiazolyl)-methylamid.

Verwendet man 3-(2,6-Dichlor-4-trifluormethyl-phenoxy)-phenoxy-essigsäurechlorid und Diäthylamin als Ausgangsstoffe, so kann der Reaktionsablauf nach dem erfindungsgemäßen Verfahren (a) durch das folgende Formelschema wiedergegeben werden:

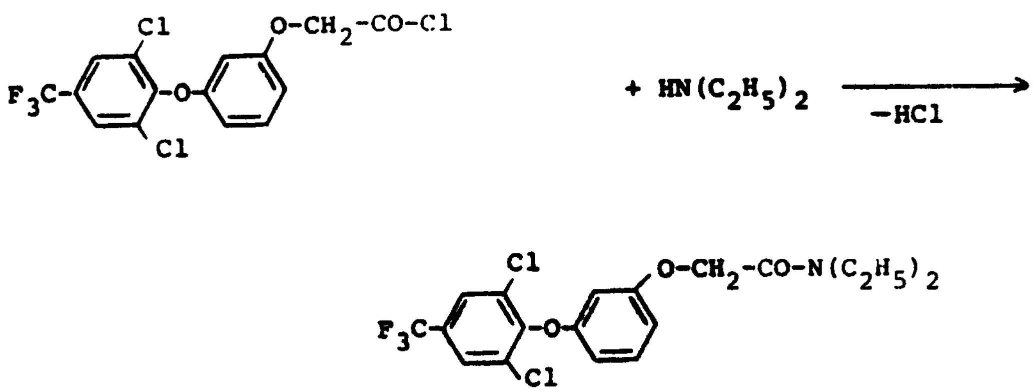


Verwendet man 2,6-Dichlor-4-trifluormethyl-3'-hydroxy-diphenyläther und Brom-essigsäure-methylester als Ausgangsstoffe, so kann der Reaktionsablauf nach dem erfindungsgemäßen Verfahren (b) durch das folgende Formelschema wiedergegeben werden:

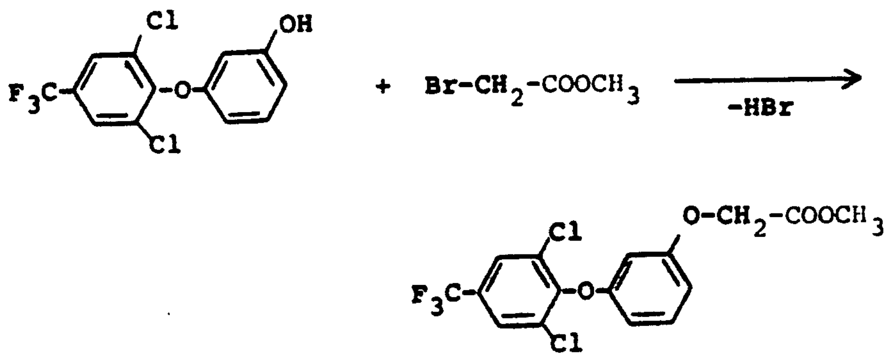


Verwendet man 3-(2,6-Dichlor-4-trifluormethyl-phenoxy)-phenoxy-essigsäure-methylester und Natriumhydroxid als Ausgangsstoffe, so kann der Reaktionsablauf nach dem erfindungsgemäßen Verfahren (c) durch das folgende Formelschema wiedergegeben werden:

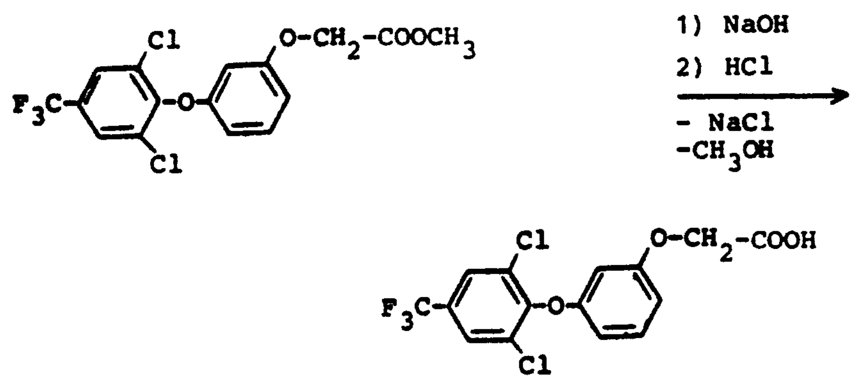


Verwendet man 3-(2,6-Dichlor-4-trifluormethyl-phenoxy)-phenoxy-essigsäure-methylester und n-Butanol als

Ausgangsstoffe, so kann der Reaktionsablauf nach dem erfindungsgemäßen Verfahren (d) durch das folgende Formelschema wiedergegeben werden:

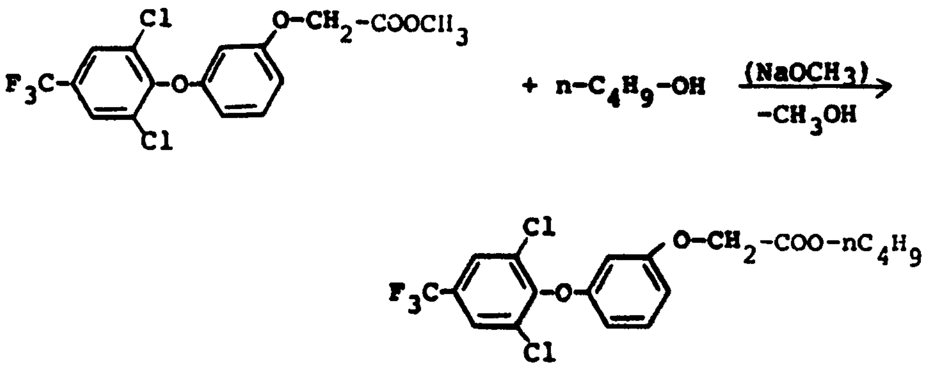


Das bei der Durchführung des erfindungsgemäßen Verfahrens (a) als Ausgangsstoff benötigte Phenoxyessigsäurechlorid ist durch die Formel (II) definiert.

Die betreffende Verbindung ist bislang noch nicht bekannt. Sie läßt sich jedoch in einfacher Weise nach im Prinzip bekannten Verfahren herstellen. So erhält man das Phenoxyessigsäurechlorid der Formel (II), indem man die entsprechende Säure der Formel

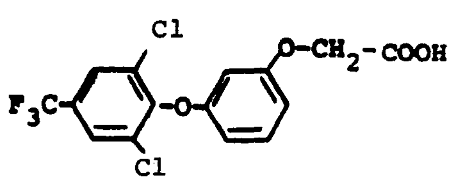
(I")

mit Chlorierungsmitteln, wie Thionylchlorid, gegebenenfalls in Gegenwart von Verdünnungsmitteln, wie Äthylenchlorid, bei Temperaturen zwischen 0°C und 50°C, vorzugsweise zwischen 10°C und 30°C umsetzt.

Die substituierte Phenoxyessigsäure der Formel (I") ist ebenfalls bisher noch nicht bekannt. Sie läßt sich jedoch ebenfalls in einfacher Weise nach im Prinzip bekannten Verfahren herstellen. So erhält man die subsitutierte Phenoxyessigsäure der Formel (I"), indem man den entsprechenden Methylester der Formel

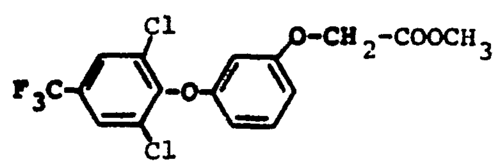

(I')

mit starken Basen, wie z.B. Natriumhydroxid oder Kaliumhydroxid, in Gegenwart eines Verdünnungsmittels, wie Methanol oder Wasser, bei Temperaturen zwischen 20°C und 100°C, vorzugsweise zwischen 40°C und 80°C behandelt.

Der substituierte Phenoxyessigsäuremethylester der Formel (I') ist ebenfalls bisher noch nicht bekannt. Er läßt sich jedoch ebenfalls in einfacher Weise nach im Prinzip bekannten Verfahren herstellen. So erhält man den substituierten Pehnoxyessigsäuremethylester der Formel (I'), indem man den Diphenyläther der Formel

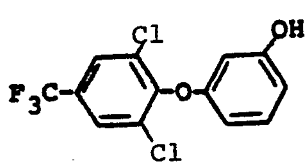

(IV)

mit Bromessigsäure-methylester der Formel

$$Br-CH_2-COOCH_3 \qquad (V)$$

in Gegenwart eines Säurebindemittels, wie Natriummethylat, Natriumäthylat oder Kaliumcarbonat, in Gegenwart eines polaren Verdünnungsmittels, wie Methanol oder Acetonitril, bei Temperaturen zwischen 20°C und 100°C, vorzugsweise 30°C und 80°C umsetzt.

Der Diphenyläther der Formel (IV) ist bisher noch nicht bekannt. Er läßt sich jedoch in einfacher Weise nach im Prinzip bekannten Verfahren herstellen. So erhält man den Diphenyläther der Formel (IV), indem man halogenierte Trifluormethyl-benzole der Formel

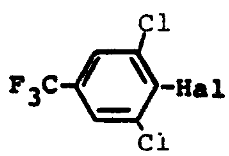

(VII)

in welcher

Hal für Chlor oder Brom steht,

mit Resorcin der Formel

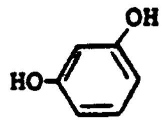

(VIII)

in Gegenwart eines Säurebindemittels sowie in Gegenwart eines Verdünnungsmittels bei Temperaturen zwischen 20°C und 200°C, vorzugsweise zwischen 50°C und 150°C umsetzt.

Als Säurebindemittel kommen hierbei alle üblichen Basen in Frage. Hierzu gehören vorzugsweise Alkalihydroxide, wie Natriumhydroxid und Kaliumhydroxid und Erdalkalihydroxide, wie Calciumhydroxid.

Als Verdünnungsmittel kommen alle üblichen inerten aprotischen polaren Lösungsmittel in Frage. Hierzu gehören vorzugsweise Amide, wie Hexamethylphosphorsäuretriamid, Dimethylformamid oder Dimethylacetamid, ferner Sulfoxide, wie Dimethylsulfoxid, weiterhin Ketone, wie Methyl-äthylketon, außerdem Nitrile, wie Acetonitril und Sulfolan.

Die halogenierten Trifluormethyl-benzole der Formel (VII) sind bekannt.

Die bei der Durchführung des erfindungsgemäßen Verfahrens (a) weiterhin als Ausgangsstoffe verwendbaren Verbindungen sind durch die Formel (III) allgemein definiert. In der Formel (III) steht R vorzugsweise für diejenigen Reste, die bereits im Zusammenhang mit der Beschreibung der erfindungsgemäßen Stoffe der Formel (I) vorzugsweise für R genannt wurden.

Die Verbindungen der Formel (III) sind bekannt. Als Beispiele seien im einzelnen genannt:

Wasser
Methanol
Äthanol
Propanol
Isopropanol
n-Butanol
tert.-Butanol
2-Methoxy-äthanol
2-Äthoxy-äthanol
2-Methylthio-äthanol
Äthoxycarbonyl-methanol
2-Äthoxycarbonyl-äthanol
2-Phenylmethylaminocarbonyl-äthanol
2-Chlor-äthanol
Ammoniak
Hydrazin
Methylamin
Äthylamin
n-Propylamin
Isopropylamin
2-Methoxy-äthylamin
2-Dimethylamino-äthylamin
Dimethylamin
Diäthylamin
Di-n-propylamin
Buten-2-yl-amin

Anilin
Phenyl-methyl-amin
4-Methyl-anilin
4-Methoxy-anilin
4-Äthoxycarbonyl-anilin
4-Chloranilin
4-Nitro-anilin
Piperidin
2-Methylpiperidin
Pyrrolidin
Piperazin
Pyrazolidin
Morpholin
2-Benzothiazolyl-methyl-amin

Als Verdünnungsmittel kommen bei der Durchführung des erfindungsgemäßen Verfahrens (a) alle inerten organischen Lösungsmittel in Betracht. Hierzu gehören vorzugsweise Ketone, wie Diäthylketon und Methyl-isobutyl-keton, ferner Nitrile, wie Acetonitril und Propionitril, weiterhin Äther, wie Tetrahydrofuran und Dioxan, darüber hinaus aliphatische und aromatische Kohlenwasserstoffe, wie Petroläther, Benzol, Toluol und Xylol, außerdem halogenierte Kohlenwasserstoffe, wie Methylenchlorid, Tetrachlorkohlenstoff, Chloroform und Chlorbenzol, außerdem Ester wie Essigester, sowie Formamide, wie Dimethylformamid.

In manchen Fällen, in denen es sich bei der Verbindung der Formel (III) um eine flüssige Komponente handelt,

erübrigt sich der Zusatz eines Verdünnungsmittels. Es kann jedoch auch in diesen Fällen ein Verdünnungsmittel hinzugefügt werden.

Als Säurebindemittel können bei der Durchführung des erfindungsgemäßen Verfahrens (a) alle üblicherweise verwendbaren anorganischen und organischen Säureakzeptoren eingesetzt werden.Hierzu gehören vorzugsweise Alkalicarbonate, beispielsweise Natriumcarbonat, Kaliumcarbonat und Natriumhydrogencarbonat, ferner niedere tertiäre Alkylamine, Aralkylamine, aromatische Amine oder Cycloalkylamine, wie z.B. Triäthylamin, Dimethylbenzylamin, Pyridin und Diazabicyclooctan.

Handelt es sich bei der Verbindung der Formel (III) um ein Amin, so kann auch dieses im Überschuß eingesetzte Amin als Säurebindemittel fungieren.

Die Reaktionstemperaturen können bei dem erfindungsgemäßen Verfahren (a) in einem größeren Bereich variiert werden. Im allgemeinen arbeitet man bei Temperaturen zwischen 20°C und 100°C, vorzugsweise zwischen 30°C und 80°C.

Bei der Durchführung des erfindungsgemäßen Verfahrens (a) setzt man auf 1 Mol an Phenoxyessigsäurechlorid der Formel (II) vorzugsweise 1 bis 2 Mol oder auch einen größeren Überschuß an einer Verbindung der Formel (III) sowie gegebenenfalls 1 bis 2 Mol an Säurebindemittel ein. - Die Isolierung der erfindungsgemäßen Stoffe der Formel (I) erfolgt nach üblichen Methoden. Im allgemeinen verfährt man in der Weise, daß man das Reaktionsgemisch nach beendeter Umsetzung entweder direkt oder nach vorherigem Einengen unter vermindertem Druck mit einem organischen Lösungsmittel, wie Toluol oder Methylenchlorid, versetzt, die organische Phase dann abtrennt, wäscht und nach dem Trocknen einengt.

Der bei der Durchführung des erfindungsgemäßen Verfahrens (b) als Ausgangsstoff verwendbare Diphenyläther ist durch die Formel (IV) genau definiert.

Man erhält diesen Stoff z.B. nach der Methode, die bereits im Zusammenhang mit der Herstellung der Ausgansprodukte für das Verfahren (a) im einzelnen beschrieben wurde.

Der bei dem erfindungsgemäßen Verfahren (b) weiterhin als Ausgangsstoff benötigte Bromessigsäure-methylester der Formel (V) ist bekannt.

Die bei der Durchführung des erfindungsgemäßen Verfahrens (b) einzuhaltenden Reaktionsbedingungen wurden ebenso wie die bei der Umsetzung zu verwendenden Säurebindemittel und Verdünnungsmittel bereits im Zusammenhang mit der Herstellung der Ausgangsstoffe für das erfindungsgemäße Verfahren (a) im einzelnen beschrieben.

Bei der Durchführung des erfindungsgemäßen Verfahrens (b) setzt man auf 1 Mol Diphenyläther der Formel (IV) vorzugsweise 1 bis 2 Mol Bromessigsäure-methylester der Formel (V) sowie 1 bis 2 Mol an Säurebindemittel ein. Die Isolierung des Reaktionsproduktes erfolgt im Falle des erfindungsgemäßen Verfahrens (b) nach üblichen Methoden. Im allgemeinen verfährt man in der Weise, daß man nach beendeter Umsetzung das Reaktionsgemisch einengt, den verbleibenden Rückstand in einem organischen Lösungsmittel, wie Methylenchlorid, aufnimmt, die Lösung filtriert, wäscht und nach dem Trocknen unter vermindertem Druck einengt.

Der bei der Durchführung des erfindungsgemäßen Verfahrens (c) als Ausgangsstoff benötigte Phenoxyessigsäure-methylester ist durch die Formel (I') genau definiert.

Der Phenoxyessigsäure-methylester der Formel (I') ist bislang noch nicht bekannt. Man erhält diesen Stoff z.B. nach der Methode, die bereits im Zusammenhang mit der Herstellung der Ausgangsstoffe für das Verfahren (a) im einzelnen beschrieben wurde.

Die bei der Durchführung des erfindungsgemäßen Verfahrens (c) einzuhaltenden Reaktionsbedingungen wurden ebenso wie die bei der Umsetzung zu verwendenden Basen und Verdünnungsmittel bereits im Zusammenhang mit der Herstellung der Ausgangsstoffe für das erfindungsgemäße Verfahren (a) im einzelnen beschrieben.

Bei der Durchführung des erfindungsgemäßen Verfahrens (c) setzt man auf 1 Mol an Phenoxyessigsäure-methylester der Formel (I') vorzugsweise 1 bis 2 Mol an Base ein. Die Isolierung des Reaktionsproduktes erfolgt im Falle des erfindungsgemäßen Verfahrens (c) nach üblichen Methoden. Im allgemeinen verfährt man in der Weise, daß man nach beendeter Umsetzung das Reaktionsgemisch in Wasser gießt, die Lösung filtriert, dann ansäuert und die dabei in fester Form anfallende Säure absaugt.

Bei der Durchführung des erfindungsgemäßen Verfahrens (d) dient derjenige Phenoxyessigsäure-methylester als Ausgangsstoff , der bereits im Zusammenhang mit der Beschreibung des erfindungsgemäßen Verfahrens (c) erwähnt wurde.

Die bei dem erfindungsgemäßen Verfahren (d) weiterhin als Ausgangsstoffe zu verwendenden Alkohole sind durch die Formel (VI) allgemein definiert. In der Formel (VI) stehr $R^1$ vorzugsweise für geradkettiges oder verzweigtes Alkyl mit 2 bis 4 Kohlenstoffatomen.

Als Beispiele für Verbindungen der Formel (VI) seien im einzelnen genannt:

Äthanol
n-Propanol
Isopropanol
i-Butanol
n-Butanol
sec.-Butanol
tert.-Butanol

Die Alkohole der Formel (VI) sind bekannt.

Bei dem erfindungsgemäßen Verfahren (d) können die im Überschuß eingesetzten Reaktionskomponenten der Formel (VI) gleichzeitig als Verdünnungsmittel fungieren. Es ist jedoch auch möglich, inerte organische Lösungsmittel als Verdünnungsmittel zu verwenden.

Die Umsetzung nach dem erfindungsgemäßen Verfahren (d) wird vorzugsweise in Gegenwart eines Alkoholates, wie z.B. Natriummethylat, durchgeführt.

Die Reaktionstemperaturen können bei dem erfindungsgemäßen Verfahren (d) innerhalb eines größeren Bereiches variiert werden. Im allgemeinen arbeitet man bei Temperaturen zwischen 20°C und 200°C, vorzugsweise zwischen 60°C und 120°C.

Bei der Durchführung des erfindungsgemäßen Verfahrens (d) setzt man auf 1 Mol an Phenoxyessigsäure-methylester der Formel (I') einen Überschuß an Alkohol der Formel (VI) sowie zweckmäßigerweise eine katalytische Menge an Alkoholat ein. - Die Isolierung der Reaktionsprodukte erfolgt nach üblichen Methoden. Im allgemeinen verfährt man in der Weise, daß man das Reaktionsgemisch nach beendeter Umsetzung einengt, den verbleibenden Rückstand mit einem organischen Lösungsmittel verrührt, die entstehende Lösung filtriert, wäscht und nach dem Trocknen einengt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Spenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Besonders geeignet sind die erfindungsgemäßen Wirkstoffe zur Bekämpfung von Unkräutern in Hafer, Weizen und Mais. Sie zeigen eine sehr gute Wirkung gegen schwer bekämpfbare Unkräuter, wie z.B. Galium.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoffimprägnierte Natur- und synthetische Stoffe und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Diemthylsulfoxid, sowie Wasser; als feste Trägerstoffe: natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin—Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden, Insektiziden und Akariziden.

Die Wirkstoffe können als solche, in Form ihrer Formulierungen oder der daraus bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Spritzen, Sprühen, Stäuben, Streuen und Gießen.

Die Anwendung ist sowohl nach dem post-emergence-Verfahren als auch nach dem pre-emergence-Verfahren möglich, sie erfolgt vorzugsweise nach dem post-emergence-Verfahren.

Die eingesetzte Wirkstoffmenge kann in größeren Bereichen schwanken. Sie hängt im wesentlichen ab von der Art des gewünschten Effektes. Im allgemeinen liegen die Aufwandmengen zwischen 0,1 und 25 kg/ha, vorzugsweise zwischen 0,25 und 10 kg/ha.

Die gute herbizide Wirksamkeit des erfindungsgemäßen Wirkstoffs (1) geht aus dem nachfolgenden Beispiel hervor:

Beispiel A

Post-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die in der Tabelle angegebenen Wirkstoffmengen pro Flächeneinheit ausgebracht werden. Die Konzentration der Spritzbrühe wird so gewählt, daß in 2000 l Wasser/ha die in der Tabelle angegebenen Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Der erfindungsgemäße Wirkstoff (1) besitzt sehr gute herbizide Eigenschaften.

Tabelle A

Post-emergence-Test

| Wirkstoff | Wirkstoff-aufwand kg/ha | Cheno-podium | Sina-pis | Galin-soga | Matri-caria | Amaran-thus | Galium | Portu-lacca | Hafer | Weizen | Mais |
|---|---|---|---|---|---|---|---|---|---|---|---|
| 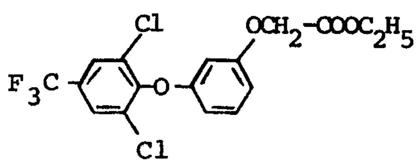 (1) | 1 | 100 | 100 | 100 | 80 | 100 | 100 | 100 | 10 | 20 | 40 |

Herstellungsbeispiele

Beispiel 1

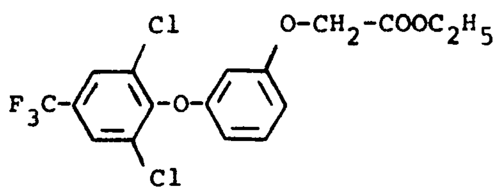

In ein Gemisch aus 24,2 g (0,075 Mol) 2,6-Dichlor-4-trifluormethyl-3'-hydroxy-diphenyläther, 12 g Kaliumcarbonat und 80 ml Acetonitril werden bei einer Temperatur von 45 bis 50°C innerhalb von 2 Stunden 13,8 g Bromessigsäure-äthylester eingetropft. Man läßt 3,5 Stunden bei 50 bis 60°C nachrühren und arbeitet dann auf, indem man das Reaktionsgemisch in Wasser gießt, das entstehende Gemisch mehrfach mit Toluol extrahiert, die vereinigten organischen Phasen trocknet und dann unter vermindertem Druck einengt. Man erhält auf diese Weise 23,7 g (77,3 % der Theorie) an 3-(2,6-Dichlor-4-trifluormethyl-phenoxy)-phenoxy-essigsäure-äthylester in Form eines organgefarbenen Feststoffes vom Schmelzpunkt 61-62°C.

Analyse:

Summenformel: $C_{17}H_{13}Cl_2F_3O_4$

| | | | |
|---|---|---|---|
| berechnet: | 49,9 % C; | 3,2 % H; | 17,4 % Cl |
| gefunden: | 49,9 % C; | 3,3 % H; | 17,1 % Cl |

Herstellung des als Ausgangsprodukt benötigten 2,6-Dichlor-4-trifluormethyl-3'-hydroxy-diphenyläthers der Formel

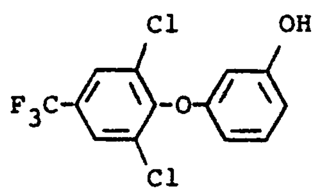

In ein Gemisch aus 330 g (3 Mol) Resorcin und 111 g (1,5 Mol) Calciumhydroxid in 2 l Dimethylsulfoxid werden bei 120-130°C innerhalb von 7 Stunden 358 g (1,5 Mol) 3,4,5-Trichlor-benzotrichlorid eingetropft. Anschließend wird die Reaktionsmischung noch 8 Stunden bei 130°C nachgerührt. Nach dem Abkühlen auf Raumtemperatur wird das Reaktionsgemisch in 7 l Wasser gegossen, wobei sich das Reaktionsprodukt ölig abscheidet. Man extrahiert mit 3 l Toluol, trennt die organische Phase ab und engt nach dem Trocknen ein. Der verbleibende Rückstand wird destilliert. Man erhält auf diese Weise 350 g (73 % der Theorie) an 2,6-Dichlor-4-trifluormethyl-3'-hydroxy-diphenyläther in Form einer Festsubstanz vom Schmelzpunkt 64-65°C.
Sdp. 123-130°C / 0,15 Torr

**Beispiel 2**

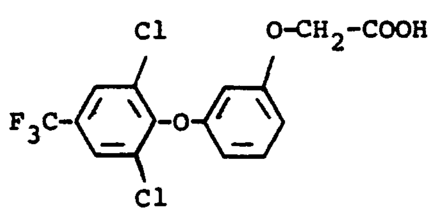

Zu 41 g (0,1 Mol) 3-(2,6-Dichlor-4-trifluormethyl-phenoxy)-phenoxy-essigsäure-äthylester und 70 ml Methanol gibt man 30 ml Wasser und 15 ml konzentrierte Natronlauge und läßt 5 Stunden bei 50 - 60°C nachrühren. Anschließend arbeitet man auf, indem man das Reaktionsgemisch in 0,5 l Wasser gießt, die Lösung heiß filtriert, ansäuert und das kristallin anfallende Produkt absaugt. Man erhält auf diese Weise 35 g (87,5 % der Theorie) an 3-(2,6-Dichlor-4-trifluormethyl-phenoxy)-α-phenoxy-essigsäure in Form einer elfenbeinfarbenen Festsubstanz vom Schmelzpunkt 104°C.

Analyse:

Summenformel: $C_{15}H_9Cl_2F_3O_4$

| | | | |
|---|---|---|---|
| berechnet: | 47,3 C; | 2,36 H; | 18,6 Cl |
| gefunden: | 47,1 C; | 2,2 H; | 17,9 Cl |

Nach einem oder mehreren der vorstehend beschriebenen Verfahren werden die in der nachfolgenden Tabelle formelmäßig aufgeführten Stoffe hergestellt.

Tabelle 1

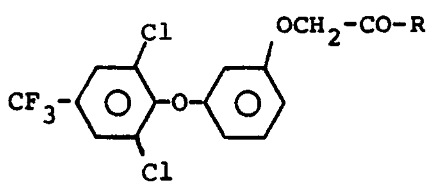

| Beispiel Nr. | R | Schmelzpunkt |
|---|---|---|
| 3 | $-O-CH_3$ | 46 - 47°C |

| Beispiel Nr. | R | Schmelz-punkt |
|---|---|---|
| 4 | $-O-C_4H_9-n$ | |
| 5 | $-O-C_4H_9-iso$ | 68°C |
| 6 | $-O-CH_2-CO_2CH_3$ | |
| 7 | $-O-CH_2-CO_2C_4H_9-n$ | |
| 8 | 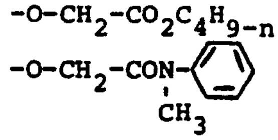 | |
| 9 | 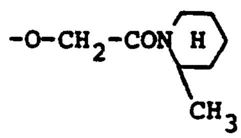 | |
| 10 | $-O-C_2H_4-S-C_2H_5$ | |
| 11 | 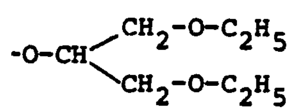 | |
| 12 | $-NH-CH_2-CH=CH_2$ | |
| 13 | 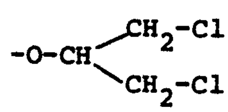 | |

| Beispiel Nr. | R | Schmelz-punkt |
|---|---|---|
| 14 | 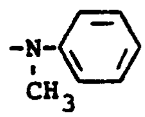 | |
| 15 | 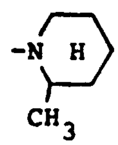 | |
| 16 | 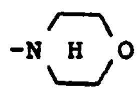 | |
| 17 | $-NH-C_2H_4-N(C_2H_5)_2$ | |
| 18 | $-N(CH_3)_2$ | |
| 19 | 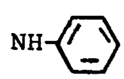 | 157°C |

Patentansprüche

1. Phenoxyessigsäure-Derivate der Formel

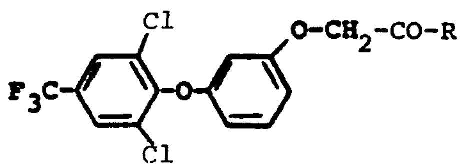

(I)

in welcher

R für Hydroxy, gegebenenfalls durch Alkoxy, Alkylthio, Carbalkoxy, Phenylmethylaminocarbonyl und/oder Halogen substituiertes Alkoxy, Amino, Hydrazino, gegebenenfalls durch Alkoxy oder Dialkylamino substituiertes Alkylamino, Dialkylamino, Alkenylamino, Arylamino oder N-Aryl-N-alkyl-amino, wobei der Arylrest jeweils durch Alkyl, Alkoxy, Carbalkoxy, Halogen und/oder Nitro substituiert sein kann, ferner für einen gegebenenfalls durch Alkyl substituierten, über Stickstoff gebundenen gesättigten heterocyclischen Ring mit 5 oder 6 Ringgliedern, oder für den Rest der Formel

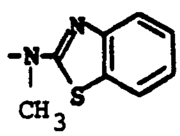

steht.

2. Verfahren zur Herstellung von Phenoxyessigsäure-Derivaten, dadurch gekennzeichnet, daß man

a) Phenoxyessigsäure-chlorid der Formel

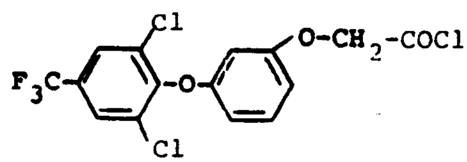

(II)

mit Verbindungen der Formel

HR (III)

in welcher

R die oben angegebene Bedeutung hat,

gegebenenfalls in Gegenwart eines Säureakzeptors sowie gegebenenfalls in Gegenwart eines Verdünnungsmittels umsetzt,

oder

b) zur Herstellung derjenigen Verbindung der Formel (I), in der R für Methoxy steht, den Diphenyläther der Formel

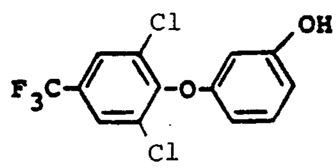

(IV)

mit Bromessigsäure-methylester der Formel

$$Br-CH_2-COOCH_3 \qquad (V)$$

in Gegenwart eines Säureakzeptors sowie in Gegenwart eines Verdünnungsmittels umsetzt,

oder

c) zur Herstellung derjenigen Verbindung der Formel (I), in der R für Hydroxyl steht, die Verbindung der Formel

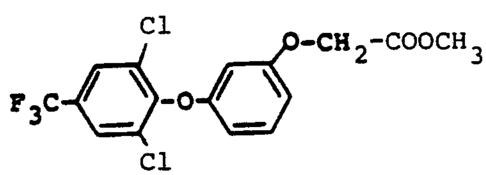

(I')

mit Basen in Gegenwart eines Verdünnungsmittels behandelt und anschließend ansäuert.

oder

d) zur Herstellung derjenigen Verbindungen der Formel (I), in denen R für Alkoxy mit mehr als einem Kohlenstoffatom steht, die Verbindung der Formel

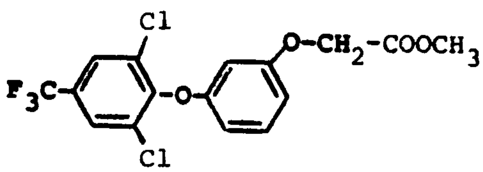

(I')

mit Alkoholen der Formel

HOR$^1$ (VI)

in welcher

$R^1$ für Alkyl mit mehr als einem Kohlenstoffatom steht,

gegebenenfalls in Gegenwart eines Alkoholates sowie gegebenenfalls in Gegenwart eines Verdünnungsmittels umsetzt.

3. Herbizide Mittel, gekennzeichnet durch einen Gehalt an mindestens einem Phenoxyessigsäure-Derivat gemäß Anspruch 1.

4. Verfahren zur Bekämpfung von Unkräutern, dadurch gekennzeichnet, daß man Phenoxyessigsäure-Derivate gemäß Anspruch 1 auf die Unkräuter und/oder deren Lebensraum einwirken läßt.

5. Verwendung von Phenoxyessigsäure-Derivaten gemäß Anspruch 1 zur Bekämpfung von Unkräutern.

6. Verfahren zur Herstellung herbizider Mittel, dadurch gekennzeichnet, daß man Phenoxyessigsäure-Derivate gemäß Anspruch 1 mit Streckmitteln und/oder oberflächenaktiven Mitteln vermischt.

7. 3-(2,6-Dichlor-4-trifluormethyl-phenoxy)-phenoxyessigsäure-chlorid.

Europäisches Patentamt

# EUROPÄISCHER RECHERCHENBERICHT

0003586
Nummer der Anmeldung
EP 79 100 332.0

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | DE - A - 2 637 098 (SOCIÉTÉ DE RECHERCHES INDUSTRIELLES ) *Anspruch 1; Seite 20, Zeile 9* -- | 1-5 | C 07 C 59/26<br>C 07 C 69/67<br>A 01 N 9/26 |
| A | DE - A - 2 643 458 (ISHIHARA SANGYO KAISHA) *Ansprüche 1, 8 und 10* -- | 1-5 | |
| D | DE - A - 2 716 189 (SOCIÉTÉ DE RECHERCHES INDUSTRIELLES) *Ansprüche 1 und 15* -- | 1-5 | RECHERCHIERTE SACHGEBIETE (Int. Cl.²)<br>A 01 N 9/26<br>C 07 C 59/26<br>C 07 C 69/00<br>C 07 C 79/35<br>C 07 C 103/00<br>C 07 C 109/08<br>C 07 C 125/06<br>C 07 C 149/00<br>C 07 D 277/82 |
| | DE - A - 2 732 442 (CIBA-GEIGY) * Ansprüche 1, 6 und 7* -- | 1-5 | |
| A,P | DE - A - 2 809 541 (CIBA GEIGY) *Ansprüche 1 und 38* ---- | 1-5 | |

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 08-05-1979 | KNAACK |